# EUROPEAN PATENT SPECIFICATION

(11) **EP 3 117 858 B1**
(45) Date of publication and mention of the grant of the patent: **26.12.2018**
(21) Application number: 16183458.5
(22) Date of filing: 01.07.2010
(51) Int. Cl.: A61M 11/00, B05B 17/06, A61M 15/00, A61M 16/00, A61M 16/06, A61M 16/20

(54) **NEBULIZER FOR INFANTS AND RESPIRATORY COMPROMISED PATIENTS**
VERNEBLER FÜR SÄUGLINGE UND PATIENTEN MIT BEEINTRÄCHTIGTEN ATEMWEGEN
NÉBULISEUR POUR NOURRISSONS ET LES PATIENTS AYANT DES VOIES RESPIRATOIRES ALTÉRÉES

(30) Priority: 01.07.2009 US 222418 P; 30.06.2010 US 828133
(43) Date of publication of application: 18.01.2017
(62) Divisional of application: 10794781.4
(73) Proprietor: MicroDose Therapeutx, Inc., Ewing, NJ 08628 (US)
(72) Inventor: GUMASTE, Anand, West Windsor, NJ 08550 (US); FLEMING, Scott, Ewing, NJ 08628 (US); CHAN, Phiip, Hightstown, NJ 08520 (US)
(74) Representative: Elkington & Fife LLP

(56) References cited:
- WO-A1-03/059424
- US-A- 5 479 920
- US-A1- 2005 229 928

## Description

This invention relates to a device and method for and dry nebulization of an aerosolizable material. The invention has particular application to delivery of powdered pharmaceutical preparations to infants and respiratory compromised patients and will be described in connection with such utility, although other utilities are contemplated.

A majority of the drugs used to treat asthma and chronic obstructive pulmonary disease (COPD) are inhaled. Recently, however, there has been a move to deliver drugs to the lungs to treat other diseases, such as diabetes, through systemic absorption. The delivery of the drug to the lungs requires that the drug be in the form of a fine aerosol suitable for inhalation. It is the opinion of the pharmaceutical industry that the particles in the aerosol need be between 1 to 5 microns in size for effective delivery and absorption. These particles in the aerosol may be either in a dry powder format or droplets of a liquid medium having the drug suspended or dissolved in it. The general advantages of pulmonary delivery are avoidance of first pass metabolism, site specific delivery of the drug, potential higher bio availability, etc. Three types of devices have been traditionally used to create the aerosol needed for pulmonary delivery- metered dose inhalers (MDis), dry powder inhalers (DPis) and aqueous nebulizers.

MDIs have a pressurized canister filled with a liquid propellant. The drug is either suspended or dissolved in the propellant. The MDIs have a metering valve for metering out a known quantity of the propellant and hence the drug. When the canister is depressed against the MDI housing a known quantity of the propellant is discharged. The propellant evaporates leaving behind a fine aerosol of the drug suitable for inhalation by the patient. For effective delivery of the drug to the lungs the patient needs to co-ordinate breath inhalation with the discharge of the drug from the canister. Patients are not always effective in achieving this co-ordination leading to dose variability. Incorporation of a breath actuation mechanism addresses this concern but the variability still exists because of the "cold" freon effect where the patient stops breathing when the cold aerosol hits the back of the throat. This is especially true of the pediatric patients where co-ordination is of major concern. To overcome these limitations and to minimize the variability ofthe dose delivered, the MDI is normally recommended to be used with a spacer especially for children. This primary function of the spacer is to slow down the MDI discharge and function as a holding chamber for the aerosol plume. A face mask may be attached to the end of the spacer. These spacers normally are made of plastic and therefore tend to build up electrostatic charge on the inside surface of the spacer. The large dead space between the inlet and outlet of the spacer coupled with the electrostatic charge has the effect of lowering the amount of dose delivered and the amount of drug that is in the respirable range. It is estimated that MDis deliver about 10% to 20% ofthe dose to lungs in adults with good co-ordination. Studies have shown that for pediatric patients between 3 years to 5 years using an MDI with a spacer and face mask, the lung delivery is <10% ofthe dose.

In DPis the drug is micronized to the right size required for pulmonary delivery. If the drug is potent it normally is mixed with an excepient such as lactose. When drugs are micronized to this size they tend to aggregate. As mentioned above, it is commonly accepted in the pharmaceutical industry that particle sizes, as a unit or in aggregate, need to be between 1 and 5 micron for effective delivery to the lungs. The aggregates are dispersed into an aerosol by introducing the drug into a strong airflow. The airflow needed to disperse the powder typically is high ranging from 30 Llmin to 90 Llmin. Failure to establish this airflow can result in a lower dose being delivered to the lungs. Any inconsistency in the breathing will lead to variability in dose delivered. As an example a so-called Turbuhaler inspiratory flow-driven inhaler has been developed and is approved for children 6 years and above delivers 20- 30% of the drug to the lungs when the airflow established by the patient is 60 Llmin. However when the airflow drops to 36 Llmin the amount of drug delivered is only 15%. The patient must therefore use rapid deep inhalation to adequately disperse the powder. This may not be possible for infants, young children and respiratory compromised patients of any age. Besides the inability of these patients to establish a strong airflow they also have low inhalation volumes. This severely impedes their ability to effectively clear the aerosol created and stored in a holding chamber such as that used by Exubera® (Nektar, San Carlos, CA).

Nebulizers, such as the jet nebulizers, produce a fine aerosol mist/droplets which carry the drug either as a suspension or dissolved in the aqueous medium. The jet nebulizers use compressed air to atomize the aqueous solution. The flow rate of the compressed air should be matched to the inhalation flow rate of the patient for optimum delivery of the drug. The patient can be administered the drug with repetitive non-forced inhalation over a prolonged period of time. The amount of drug delivered is influenced by a large number of factors such as viscosity, volume of drug fill, surface tension, inhalation flow, etc. The amount of drug delivered ranges from 3% to 6% for pediatric patients and 3% to 13% for adults. For pediatric delivery the nebulizers are normally coupled to a face mask. Since the nebulizer continues to produce the aerosol during the exhale cycle of the breath this leads to drug wastage, increased exposure ofthe drug to the patient's face and eyes and also to the care-giver. The disadvantages of nebulizers in general are their poor efficiency of delivery to the patient, a requirement for a compressor or compressed air and long delivery times, on the order of 5 to 15 minutes, etc.

Thus there is a need for a delivery mechanism for infants and young children, and also for respiratory compromised patients that overcomes the aforesaid and other disadvantages of the prior art, in a manner that delivers the drug efficiently, does not require inhalation co-ordination, operates under low inhalation volume, minimizes the exposure of the care giver to the drug, delivers the drug in a short time (preferably less than a minute), and is low cost and portable.

The invention is defined by the appended claims. The present invention provides a device, its use and method for aerosolized dosing of dry powder pharmaceutical preparations, which overcomes the aforesaid and other problems of the prior art, and provides a simple and relatively low cost device operative independently of a source of compressed carrier air. More particularly, in accordance with the present invention there is provided a device, its use and method for aerosolized dosing of dry powder pharmaceutical preparations, or pharmaceutical agents dissolved or suspended in a liquid medium comprising a pharmaceutical aerosolization engine comprising a vibratory device. In one embodiment, the aerosolization engine is connected to a face mask and permits manual activation of the aerosolization engine by a caregiver, and presentation of aerosolized medication into the face mask. The face mask may be replaced with a nasal cannula or a mouth piece and the manual activation may be replaced with automated activation of the aerosolization engine through sensing of the patients' inhalation or tidal breathing maneuver, or through synchronization with hospital equipment operating to assist or substitute for the patient's breathing as in ventilators or in delivering oxygen or humidified air for example. The present invention has particular utility in connection with aerosolization and delivery of dry powdered pharmaceutical agents to an infant or small child and will be described in connection with such utility, although other utilities including continuous or semi-continuous or intermittent nebulization of dry powder pharmaceutical agents, pharmaceutical agents dissolved or suspended in a liquid medium, and delivery to infants and small children, and to respiratory compromised patients, ventilated patients and unconscious patients is also contemplated.

WO 03/059424 A1 relates to methods and systems for operating an aerosol generator.

Features and advantages of the present invention will be seen from the following detailed description, taken into conjunction with the accompanying drawings, wherein:
Fig. 1 is a perspective view of a hand-held pediatric nebulizer in accordance with the preferred embodiment of the invention;
Fig. 2 is a top plan view of the device of Fig. 1;
Fig. 3 is a bottom plan view showing details of the facemask portion of the device ofFig. 1;
Fig. 4 is a schematic diagram illustrating generation of nebulized powder medication in accordance with the present invention;
Fig. 5 is a perspective view illustrating a pharmaceutical package in accordance with a preferred embodiment of the invention;
Fig. 6 is a flow diagram illustrating another embodiment of the invention; and
Fig. 7 is a schematic of the timing diagram for the intermittent excitation of the aerosol engine.

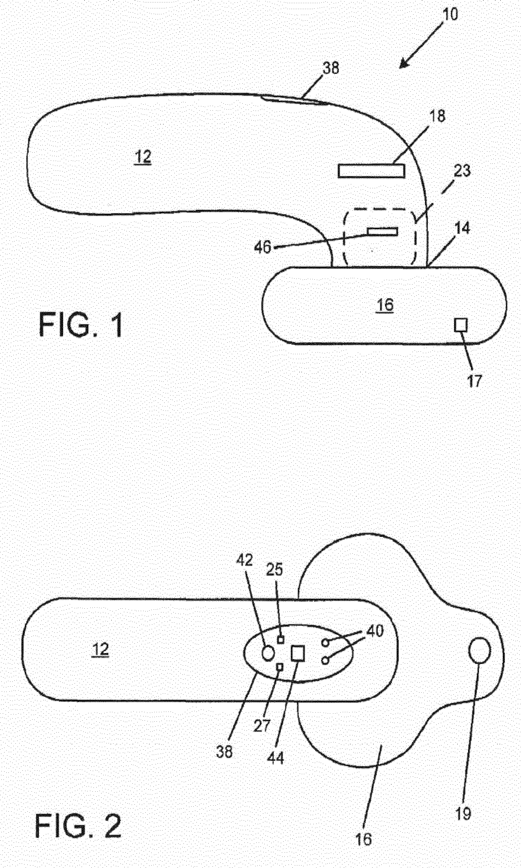

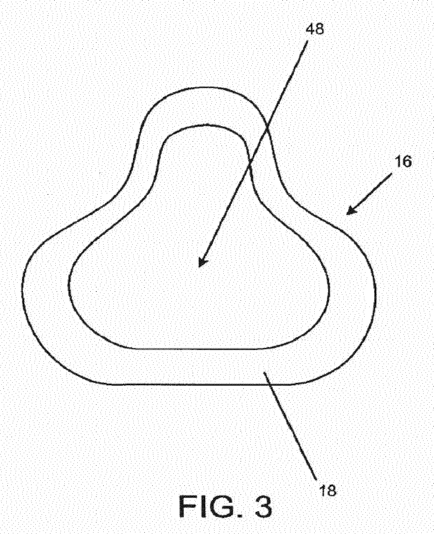

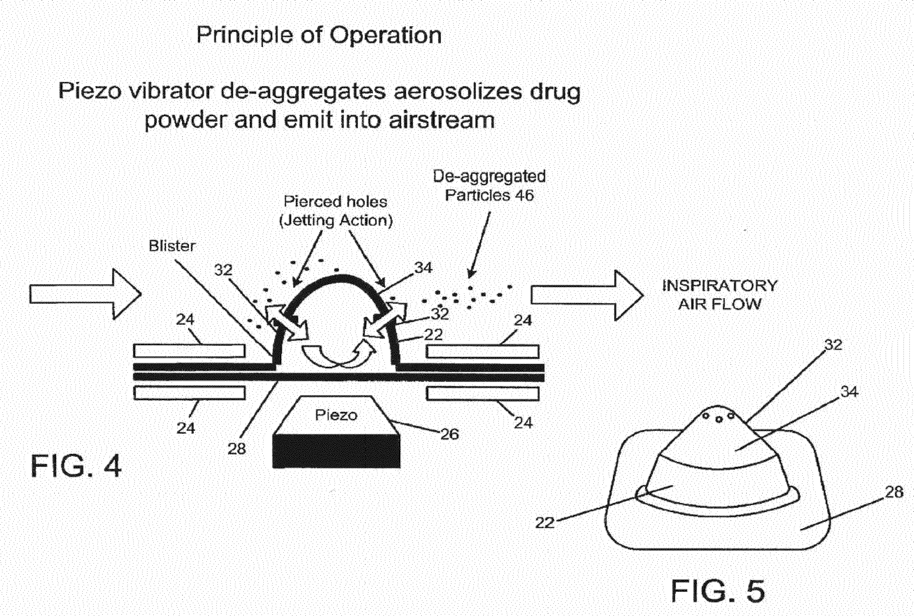

Turning now to Figs. 1-5 of the drawings, there is illustrated a dry powder pediatric nebulizer in accordance with the present invention. The nebulizer 10 comprises a housing or body 12 sized and shaped to fit comfortably within the hand of a human adult. Body 12 houses a dry powder aerosol engine, battery power and controls all as will be discussed below. Referring in particular to Figs. 2 and 3, the hand held nebulizer 10 is connected at its outlet 14 to a facemask 16. Facemask 16 is sized and shaped to fit over the mouth and nose of a patient, and is formed of a resiliently deformable material such as silicon rubber. Facemask 16 may comprise a single wall construction or, if desired may comprise a soft partially air-filled cuff 18 at its distal end, and optionally may include a one-way filter valve 19 to allow the patient's exhale breath to escape. Facemask 16 is friction fitted to the outlet end of nebulizer device 12 so that it may be removed for cleaning and/or disposal and a fresh facemask placed thereon. Also, if desired, facemask 16 may come in different sizes, e.g. for adults, children and infants. The face mask may incorporate a pressure sensor 17 to measure the quality of fit and seal over the patient or the sensor may be incorporated into the inhaler housing. A good seal is preferred to ensure high efficiency of delivery of the drug to the patient and to protect the care-giver from exposure to the drug and the patient from exposure of the drug to the eyes.

Referring also to Figs. 3-5 body 12 includes a movable panel 18 for permitting one or more blister packs or molded bodies 22 containing a powdered medication to be introduced into a chamber 23 (shown in phantom) defined within the interior of body 12. Blister pack 22 is guided by guides 24 to locate in contact with the top surface of an aerosolization engine in the form of a vibratory element 26. Alternatively, body 22 may be a molded body that is reused over a number of closings. The body in this case provides a way for introducing the drug into the chamber. Vibratory element 26 preferably comprises a piezo activator or piezo transducer or a mechanical vibrator, an electro-mechanical vibrator or a magnetostrictive element or other vibratory mechanism. Preferred are aerosolization engines and aerosolization chambers such as described in U.S. Patent Nos. 6,026,809, 6,142,146, 6,152,130, 7,318,434, 7,334,577, 7,343,914 and published U.S. Application Nos. 2005/0172962 and 2008/0202514.

Blister pack 22 preferably comprises a domed dry powder drug package made of cold formed or thermal formed film, and includes a conical, semi-spherical, elliptical, pyradidal or similar top part 34 and flat base 28 such as described in U.S. 7,080,644, assigned to the common assignee. Blister pack 22 has at least one drug ejection aperature 32 substantially opposite base 28 and serving primarily for injection of drug particles. Aperatures 32 may be pre-formed integrally with capsule 22, or formed as puncture holes when the capsule 22 is inserted into body 12.

Blister pack 22 carries a supply of a drug substance or substances which preferably are provided as a dry powder. A single component or several drug combinations may be used, or, the drug substance or substances combined with excipients, such as lactose or combinations thereof. Other additives such as pharmaceutically inactive ingredients, de-aggregation agents, etc., also may be added.

Body 12 carries a battery 25 for powering the vibratory element 26, as well as a microprocessor or electronic controller 27 for controlling operation of the vibratory element 36, sensor signal processing for inhalation and/or exhalation detection,etc. Body 12 also includes a control panel 38 including one or more activation buttons 40, 42, and a display 44. The display 44 may incorporate active dose feedbacks to indicate such things as device readiness, face mask seal integrity, activation of the aerosol engine during inhalation or tidal breathing and dosing complete, such as described in U.S. Published Application No. US2005/0183725 A1. Body 12 also includes one or more side walled aperatures 46 which permit air to enter chamber (shown in phantom at 23) from the outside.

Operation of the nebulizer is as described below.

A caregiver places the facemask over the mouth and nose of the patient. Thereafter, the caregiver presses the start button 40 which activates the vibrating element 26 for a predetermined time, e.g. 1-2 seconds. The vibrating element engages with the base of blister pack 22 whereupon powdered medication is deaggregated and ejected out of blister pack 34 into chamber 23 as a cloud or powder plume 46 where it is then inhaled by the patient.

The present invention has several advantages over the prior art. For one, the ability to aerosolize dry powders and deliver same in a nebulizer permits much higher dose concentrations than are possible with liquid carried drugs. Thus, administration time for a dose may be significantly reduced over those of a liquid nebulizer. Also, many drugs are insoluble in water and can't be delivered using conventional nebulizers, or are soluble only in organic solvents which create other problems.

Another feature and advantage of the present invention is that the generation of powder plume is independent of inhalation rate and inhalation timing. Thus, the nebulizer of the present invention is particularly useful in the case of infants and small children, respiratory compromised patients, and unconscious patients. The above described invention provides controlled, reproducible and recordable pulmonary doses from pre-measured blister packs. Alternatively, a plurality of blister packs may be mounted in the body 12 as a cartridge, and advanced, as necessary. Alternatively the dose amount may be controlled by the number and duration of the delivery 'pulses', or aerosol activation cycles.

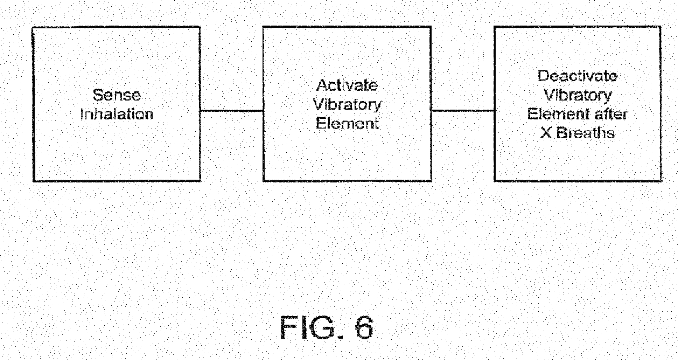

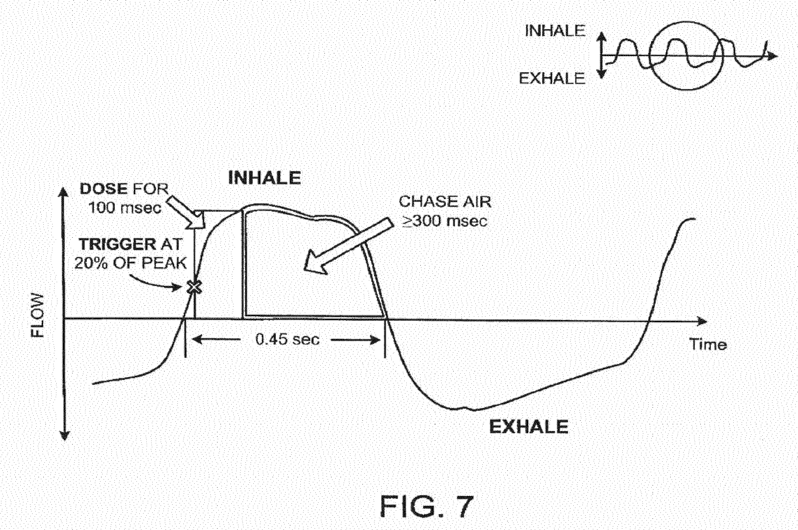

The invention is susceptible to modification. For example, facemask 16 may be removed, or the nebulizer mounted directly to a pre-existing ventilator/nebulizing system where it may be run continuously or semi-continuously or intermittedly. The nebulizer also may be triggered to turn on and off by sensing tidal breathing of a patient as illustrated in Fig. 6 and 7, and operate over one or several breaths. As shown in Figure 7 the inhalation and/or exhalation cycle is sensed and the aerosol generator is turned on for a short duration followed by an amount of chase air to carry or follow the particles into the patient. A sufficient quantity of chase air is necessary to ensure lung deposition when inhalation volumes are low and inhalation cycles are short. Any sensor or combination of sensors that can be used to measure or identify the difference in properties between an inhalation and exhalation manuever can be used to synchronize and turn the aerosol generator on and off. Example of sensors that may be used to detect the patients inhalation/exhalation are flow sensors, pressure sensors, temperature sensors that measure the temperature difference between the inhaled and exhaled breath, carbon dioxide or nitric oxide or other gas sensors that measure the gas component level difference between inhaled and exhaled breath, and also physical measurement systems such as chest straps to measure the expansion and contraction ofthe chest cavity, etc., can be employed for this purpose. Still other changes are possible. For example, active visual, audible or tactile feedback to the patient or caregiver indicating the status of the device and of dosing may be provided including, for example, visual or audible devices as taught in U.S. Patent 7,343,914. Also, if desired, electronic communication may be provided for connecting the device to equipment connected to the patient for controlling or synchronizing the vibratory element. Also, if desired, the dose or amount delivered to a patient may be determined by the counting and controlling number of timed or pulsed activations of the vibratory element. Also animal or cartoon images may be printed on the inside surface 48 of the facemask 16, to make the instrument more friendly to a child patient, or the device feedback systems, e.g. lights and sounds and vibrations may be used for this purpose.

Also, while the invention has been described in particular for use with drugs for treating asthma and COPD, the invention also advantageously may be used for delivery of other drugs including, but not limited to, anti-virals to treat viruses including but not limited to RSV, and anti-biotics, anti-fungals and anti-infectives for treating lung infections and other diseases, or drugs for treating lung cancer.

Still other changes are possible. For example, it is possible to control the amount of drug delivered to the nasal passages as opposed to just the lower respiratory track by controlling particle size. Still other changes are possible.

## Claims

1. A device for delivering dry powder medication to infants and respiratory-compromised patients comprising a housing (12) defining an aerosol chamber (23) into which an aerosolized medication may be introduced;
an outlet (14) for said chamber (23) ;
a patient interface selected from a nasal cannula or a mouthpiece connected to the outlet, or a mask (16) surrounding the outlet (14), at least in part; and
a nebulizer (26) for aerosolizing a medication and introducing the medication into the chamber (23),
wherein an inhalation and/or exhalation cycle is sensed, using sensors selected from flow sensors, pressure sensors, temperature sensors, gas sensors and chest straps, and the nebulizer (26)
is turned on and off synchronized to tidal breathing of the patient, and
wherein the medication is introduced from a dose-controlled blister pack (22),
said device further comprising an active visual, audible or tactile feedback display (44) for the patient or caregiver indicating a status of the device and of dosing, and a start button (40)
permitting a caregiver to activate the nebulizer independent of inhalation rate and timing,
wherein the nebulizer (26) comprises an aerosol generator in the form of a vibratory element, wherein during one inhalation the vibratory element turns on for a predetermined amount of time, and then remaining chase air carries medication particles into the patient.

2. The device of claim 1, **characterized in that** during one inhalation the vibratory element turns on for 100 msec, and remaining chase air carries medication particles into the patient.

3. The device of claim 1, **characterized in that** a plurality of blister packs (22) are mounted in the housing (12).

4. The device of claim 1, wherein the dose delivered amount is determined by the number of timed or pulsed activations of the vibratory element.

5. The device of claim 1, **characterized in that** the mask (16) is formed of a resiliently deformable material, and further comprising a pressure sensor for measuring the quality of fit of the mask to the patient.

## Patentansprüche

1. Vorrichtung zum Abgeben eines Trockenpulvermedikaments an Säuglinge und Patienten mit beeinträchtigten Atemwegen, die ein Gehäuse (12), das eine Aerosolkammer (23) definiert, in die ein aerosoliertes Medikament eingeführt werden kann, umfasst;
einen Auslass (14) für die Kammer (23);
eine Patientenschnittstelle, die aus einer Nasenkanüle oder einem Mundstück, das mit dem Auslass verbunden ist, oder eine Maske (16), die den Auslass (14) zumindest zum Teil umgibt, ausgewählt ist; und
einen Vernebler (26) zum Aerosolieren eines Medikaments und Einführen des Medikaments in die Kammer (23), wobei ein Inhalations- und/oder Exhalationszyklus durch Verwenden von Sensoren erfasst wird, die aus Strömungsfühlern, Drucksensoren, Temperatursensoren, Gassensoren und Brustriemen ausgewählt sind, und der Vernebler (26) synchron zur Ruheatmung des Patienten ein- und ausgeschaltet wird, und wobei das Medikament aus einer dosisgeregelten Blisterpackung (22) eingeführt wird, wobei die Vorrichtung ferner eine aktive visuelle, akustische oder haptische Feedback-Anzeige (44) für den Patienten oder die Pflegekraft, die einen Status der Vorrichtung und der Dosierung angibt, und eine Starttaste (40), die es einer Pflegekraft ermöglicht, den Vernebler unabhängig von Inhalationsrate und -zeitpunkt zu aktivieren, umfasst, wobei der Vernebler (26) einen Aerosolgenerator in Form eines Vibrationselements umfasst, wobei sich das Vibrationselement während einer Inhalation für einen vorbestimmten Zeitraum einschaltet und nachfolgend die verbleibende einströmende Luft die Medikamentenpartikel in den Patienten trägt.

2. Vorrichtung nach Anspruch 1, **dadurch gekennzeichnet, dass** während einer Inhalation das Vibrationselement sich für 100 ms einschaltet und die verbleibende einströmende Luft die Medikamentenpartikel in den Patienten trägt.

3. Vorrichtung nach Anspruch 1, **dadurch gekennzeichnet, dass** eine Vielzahl von Blisterpackungen (22) in dem Gehäuse (12) angebracht ist.

4. Vorrichtung nach Anspruch 1, wobei die abgegebene Dosismenge von der Anzahl der zeitlich abgestimmten oder gepulsten Aktivierungen des Vibrationselements bestimmt ist.

5. Vorrichtung nach Anspruch 1, **dadurch gekennzeichnet, dass** die Maske (16) aus einem elastisch verformbaren Material gebildet ist und wobei ferner ein Drucksensor zum Messen der Passqualität der Maske am Patienten umfasst ist.

## Revendications

1. Dispositif pour la distribution d'un médicament sous forme de poudre sèche à des nourrissons ou des patients souffrant de troubles pulmonaires, comprenant un boîtier (12) définissant une chambre d'aérosol (23) dans laquelle un médicament à l'état d'aérosol peut être introduit ;
une sortie (14) pour ladite chambre (23) ;
une interface patient sélectionnée parmi les suivantes : une canule nasale ou un embout raccordé à la sortie, ou un masque (16) entourant la sortie (14), au moins en partie ; et
un nébuliseur (26) pour vaporiser un médicament et l'introduire dans la chambre (23),
un cycle d'inhalation et/ou d'exhalation étant détecté, en utilisant des détecteurs sélectionnés parmi les suivants : détecteurs de flux, détecteurs de pression, détecteurs de température, détecteurs de gaz et sangles de poitrine, et le nébuliseur (26) étant activé/désactivé en synchronisation avec la respiration courante du patient,
et
le médicament étant introduit depuis une plaquette alvéolée à dose contrôlée (22),
ledit dispositif comprenant en outre un affichage de réponse visuel, audible ou tactile (44) pour le patient ou le soignant, indiquant un statut du dispositif ou de la dose, et
un bouton de marche (40) permettant au soignant d'activer le nébuliseur indépendamment du débit d'inhalation et de la synchronisation,
le nébuliseur (26) comprenant un générateur d'aérosol sous forme d'un élément vibratoire, de sorte qu'au cours d'une inhalation, l'élément vibratoire s'active pendant une période prédéterminée, puis l'air restant transportant le médicament jusqu'au patient.

2. Dispositif selon la revendication 1, **caractérisé en ce qu'**au cours d'une inhalation l'élément vibratoire s'active pendant 100 msec, et l'air restant transportant le médicament jusqu'au patient.

3. Dispositif selon la revendication 1, **caractérisé en ce qu'**une pluralité de plaquettes alvéolées (22) sont montées dans le boîtier (12).

4. Dispositif selon la revendication 1, la dose délivrée étant déterminée par le nombre d'activations temporisées ou pulsées de l'élément vibratoire.

5. Dispositif selon la revendication 1, **caractérisé en ce que** le masque (16) est réalisé avec un matériau élastiquement déformable, et comprenant en outre un capteur de pression pour mesurer la qualité d'ajustement du masque sur le patient.
